# EUROPEAN PATENT APPLICATION

(11) **EP 4 434 434 A1**
(43) Date of publication of application: **25.09.2024**
(21) Application number: 21964732.8
(22) Date of filing: 18.11.2021
(51) Int. Cl.: A61B 1/045

(54) **INFORMATION PROCESSING DEVICE, INFORMATION PROCESSING METHOD, AND RECORDING MEDIUM**

(71) Applicant: NEC Corporation, 108-8001 Tokyo (JP)
(72) Inventor: KAMIJO, Kenichi, Tokyo 108-8001 (JP); OHTSUKA, Shota, Tokyo 108-8001 (JP); KIMURA, Tatsu, Tokyo 108-8001 (JP); MARUGAME, Atsushi, Tokyo 108-8001 (JP); SAIKOU, Masahiro, Tokyo 108-8001 (JP); SHINO, Ryosaku, Tokyo 108-8001 (JP)
(74) Representative: Betten & Resch
(86) International application number: PCT/JP2021/042366
(87) International publication number: WO 2023/089717

(57) **Abstract**

The endoscopic image acquisition means acquires an endoscopic image. The still image acquisition means acquires a plurality of still images obtained by imaging a lesion included in the endoscopic image. The identity determination means determines an identity of the lesion included in the plurality of still images. The selection means selects a representative image that best represents the lesion, from a plurality of still images determined to correspond to the same lesion, and displays the representative image on the display device.

## Description

### TECHNICAL FIELD

The present disclosure relates to processing of information relating to an endoscopic examination.

### BACKGROUND ART

During an endoscopic examination, the doctor sometimes captures dozens of images. If the number of images was large, it takes time to select images to be attached to the examination report from among them. In addition, there is AI (Artificial Intelligence) for qualitative determination of a suspected portion of lesion, but when AI performs the determination process each time the doctor captures the suspected portion of lesion, the confirmation of the determination result becomes complicated and the processing load of AI also increases. For example, Patent Document 1 proposes a method that selects and outputs images of interest from among the time-series image group taken along the luminal cavity or the like of the subject in time series.

### PRECEDING TECHNICAL REFERENCES

### PATENT DOCUMENT

Patent Document 1: Japanese Patent Application Laid-Open under No. JP 2011-024727

### SUMMARY

### PROBLEM TO BE SOLVED

When multiple images captured by the doctor are related to the same lesion at the same site, it is desirable to narrow down the number of images and use them as attached images of the report or images for qualitative determination.

It is an object of the present disclosure to provide an information processing device capable of selecting the images to be attached to the report or the images for qualitative determination of AI from among a huge number of images captured in the endoscopic examination.

### MEANS FOR SOLVING THE PROBLEM

According to an example aspect of the present disclosure, there is provided an information processing device comprising:
an endoscopic image acquisition means configured to acquire an endoscopic image;
a still image acquisition means configured to acquire a plurality of still images obtained by imaging a lesion included in the endoscopic image;
an identity determination means configured to determine an identity of the lesion included in the plurality of still images; and
a selection means configured to select a representative image that best represents the lesion, from a plurality of still images determined to correspond to the same lesion.

According to another example aspect of the present disclosure, there is provided an information processing method comprising:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.

According to still another example aspect of the present disclosure, there is provided a recording medium storing a program, the program causing a computer to execute processing of:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.

### EFFECT

According to the present disclosure, it is possible to select the images to be attached to the report or the images for qualitative determination of AI from among the images captured in the endoscopic examination.

### BRIEF DESCRIPTION OF THE DRAWINGS

[FIG. 1] FIG. 1 is a block diagram showing a schematic configuration of an endoscopic examination system.
[FIG. 2] FIG. 2 is a block diagram showing a hardware configuration of an image processing device.
[FIG. 3] FIG. 3 is a block diagram showing a functional configuration of the image processing device.
[FIG. 4] FIG. 4 schematically shows a structure of a large intestine.
[FIG. 5] FIG. 5 shows a display example of an examination report.
[FIG. 6] FIG. 6 shows another display example of the examination report.
[FIG. 7] FIG. 7 is a flowchart of display processing by an image processing device.
[FIG. 8] FIG. 8 is a block diagram showing a functional configuration of an information display device of a second example embodiment.
[FIG. 9] FIG. 9 is a flowchart of processing by the information display device of the second example embodiment.

### EXAMPLE EMBODIMENTS

Preferred example embodiments of the present disclosure will be described with reference to the accompanying drawings.

### <First example embodiment>

### [System Configuration]

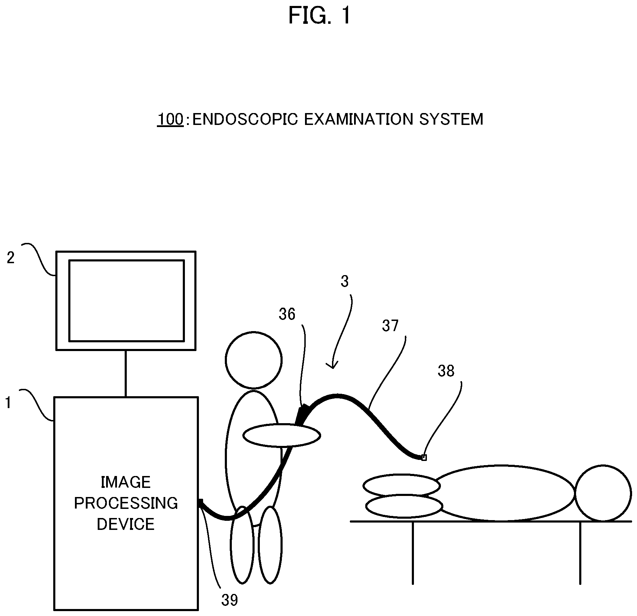

FIG. 1 shows a schematic configuration of an endoscopic examination system 100. The endoscopic examination system 100 acquires images captured by the examiner of the endoscopic examination and images captured by AI at the time of examination (including treatment) using the endoscope. Then, if there are multiple images of the same lesion among the images captured by the examiner or AI, the endoscopic examination system 100 selects a representative image from the images and displays the result. In particular, the endoscopic examination system 100 of this example embodiment is characteristic in that it selects an image that best represents the lesion as the representative image and displays the result. This eliminates the labor of selecting an image to be attached to the examination report, and makes it possible for the examiner to efficiently create the examination report.

As shown in FIG. 1, the endoscopic examination system 100 mainly includes an image processing device 1, a display device 2, and an endoscope 3 connected to the image processing device 1.

The image processing device 1 acquires a moving image (hereinafter also referred to as an "endoscopic video Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as an endoscopic video Ic during the endoscopic examination. In addition, when the examiner finds a lesion during the endoscopic examination, he or she operates the endoscope 3 to input a photographing instruction of the lesion position. Based on the photographing instruction by the examiner, the image processing device 1 generates a lesion image capturing the lesion position. Specifically, the image processing device 1 generates the lesion image which is a still image, from the endoscopic image Ic which is a moving image, on the basis of the photographing instruction of the examiner. At this time, if the generated lesion image is not clear, the image processing device 1 may notify the examiner to input the photographing instruction again.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

While the following explanation is mainly given on the processing of endoscopic examination for a large intestine, the subjects of examination may be gastrointestinal (digestive organs) such as the stomach, esophagus, small intestine, and duodenum, as well as the large intestine.

In addition, the portion to be detected in the endoscopic examination is not limited to the lesion portion, but may be any portion in which the examiner needs attention (also referred to as a "target portion"). Such a target portion may be, for example, the lesion portion, a portion where irritation has occurred, a portion where a surgical wound or other cut has occurred, a portion where folds or protrusions has occurred, or a portion where the tip portion 38 of the endoscope 3 is likely to contact (easily stuck) at the wall surface of the intraluminal region.

### [Hardware configuration]

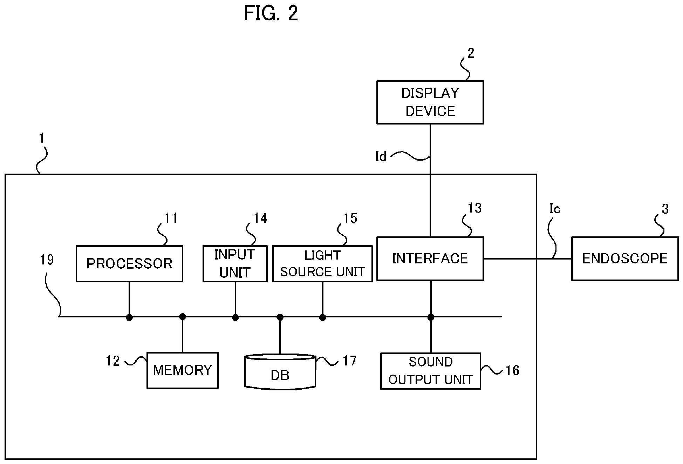

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic videos Ic taken by the endoscope 3 in the endoscopic examination, based on the control of the processor 11. Further, the memory 12 temporarily stores the lesion images photographed in response to the photographing instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic video Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic images acquired by past endoscopic examinations of the subject, and the lesion information. The lesion information includes lesion image and related information. The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

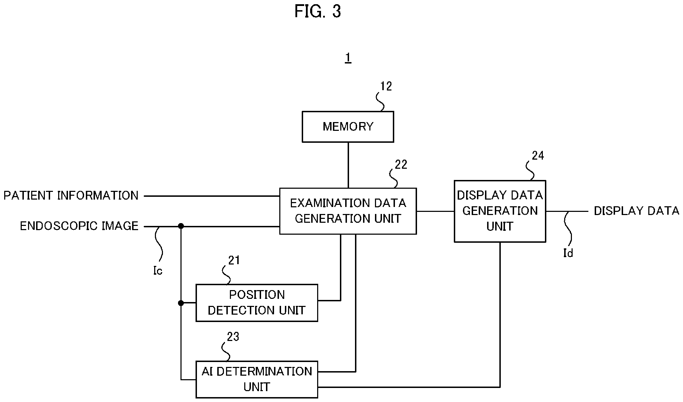

FIG. 3 is a block diagram showing a functional configuration of the image processing device 1. The image processing device 1 functionally includes a position detection unit 21, an examination data generation unit 22, an AI determination unit 23, and a display data generation unit 24.

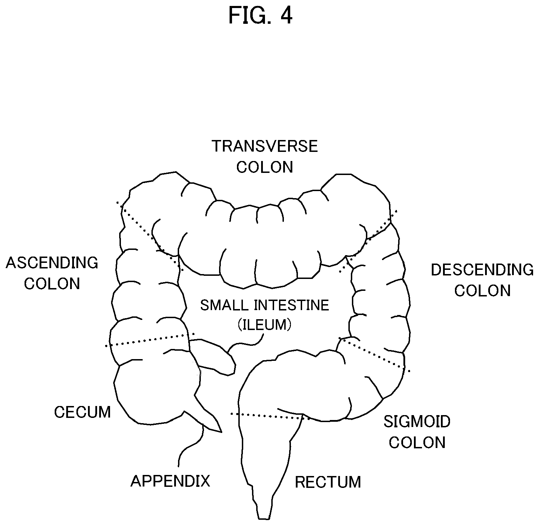

The image processing device 1 receives the endoscopic image Ic from the endoscope 3. The endoscopic image Ic is inputted into the position detection unit 21, the examination data generation unit 22, and the AI determination unit 23. The position detection unit 21 detects the position of the endoscope 3, i.e., the imaging position of the endoscopic image, based on the endoscopic image Ic. Specifically, the position detection unit 21 detects the imaging position by image analysis of the inputted endoscopic image Ic. Here, the imaging position may be three-dimensional coordinates in the organ of the examination target, but may be at least an information indicating one of a plurality of regions in the organ of the examination target. For example, as shown in FIG. 4, the large intestine includes multiple regions (sites) such as the cecum, the ascending colon, the transverse colon, the descending colon, the sigmoid colon, and the rectum. Therefore, when the examination target is the large intestine, the position detection unit 21 may detect whether at least the imaging position belongs to any of the above-described regions.

Specifically, the position detection unit 21 can estimate which region of the large intestine the imaging position at that time belongs to, based on the pattern of the mucous membrane, the presence or absence of the folds, the shape of the folds in the endoscopic image, the number of the folds passed by the movement of the endoscope 3, and the like. The position detection unit 21 may estimate the imaging position by estimating the movement speed of the endoscope 3 based on the endoscopic image and calculating the movement distance in the large intestine based on the movement speed and the time. In addition to the image analysis of the endoscopic image, the position detection unit 21 may detect the imaging position using the insertion length of the endoscope 3 inserted into the organ. In the present example embodiment, the detection method of the imaging position in the organ of the examination target is not limited to a specific method. The position detection unit 21 outputs the detected imaging position to the examination data generation unit 22.

Further, the image processing device 1 receives patient information of the patient who is the subject, through the input unit 14. The patient information is information that uniquely identifies the patient, and may be patient name, an ID uniquely assigned to each patient or a personal identification number such as a My Number. The patient information is inputted to the examination data generation unit 22.

The examination data generation unit 22 generates the examination data of the imaging position based on the endoscopic image Ic and the imaging position detected by the position detection unit 21, and temporarily stores the generated examination data in the memory 12. Specifically, when the examiner operates the endoscope 3, the examination data generation unit 22 generates the examination data including the endoscopic image Ic captured by the endoscope 3 and imaging information including the patient name, the patient ID, the examination date and time, the imaging position, and the like, and stores the examination data in the memory 12. The examination data generation unit 22 outputs the generated examination data to the AI determination unit 23, and the display data generation unit 24.

The AI determination unit 23 performs image analysis on the endoscopic image Ic to determine whether or not lesions exist. The AI determination unit 23 detects lesion-like portions included in the endoscopic image using an image recognition model prepared in advance. When detecting the lesion-like portion, the AI determination unit 23 outputs the position (lesion position) and a score indicating probability of lesion as a determination result. On the other hand, when the AI determination unit does not detect any lesion-like portion, it outputs the determination result indicating that there is no lesion.

Further, the AI determination unit 23 acquires the lesion image which is the still image generated on the basis of the photographing instruction of the examiner from the examination data generated by the examination data generation unit 22. Then, the AI determination unit 23 groups the images related to the same lesion from a plurality of lesion images. Specifically, the AI determination unit 23 performs pattern matching of the lesion portion for the plurality of lesion images captured within a predetermined period, and determines whether or not the images are related to the same lesion. The AI determination unit 23 groups the images when it is determined that the image are related to the same lesion. The AI determination unit 23 may determine whether or not the images are related to the same lesion using the above-described pattern matching and the position of the lesion.

Furthermore, the AI determination unit 23 selects the representative image from the grouped image group. Here, the representative image is the image which best represents the lesion among the grouped image group. Specifically, the AI determination unit 23 selects the representative images using the criteria exemplified below. In the first example, the AI determination unit 23 selects the image in which the lesion is the largest as the representative image among the grouped image group. In the second example, the AI determination unit 23 selects the image in which the lesion is in the most center as the representative image among the grouped image group. In the third example, the AI determination unit 23 selects the image in which the focus of the image is the most in focus as the representative image among the grouped image group. In the fourth example, the AI determination unit 23 selects the image having the highest score of lesion likelihood as the representative image among the grouped image group. The AI determination unit 23 may select the representative image by using the criterion combining these criteria.

The representative image is not limited to one. The examiner may capture the lesion image using different types of illumination light during endoscopic examination. Thus, for example, when the examiner captures the same lesion by switching the illumination light of the endoscope from white light to special light, etc., the representative image may be selected for each type of light.

The AI determination unit 23 outputs the selected representative image to the display data generation unit 24. At this time, The AI determination unit 23 performs qualitative determination for the representative image, and may output the result of the qualitative determination to the display data generation unit 24.

Incidentally, the lesion image is not limited to the still image generated on the basis of the photographing instruction of the examiner, and it may be the still image which the AI determination unit 23 generated on the basis of the endoscopic image Ic by estimating the lesion portion by itself.

The display data generation unit 24 generates the display data Id using the examination data inputted from the examination data generation unit 22 and the representative image data inputted from the AI determination unit 23, and outputs the display data to the display device 2.

### [Display example]

Next, a display example by the display device 2 will be described.

### (Examination report)

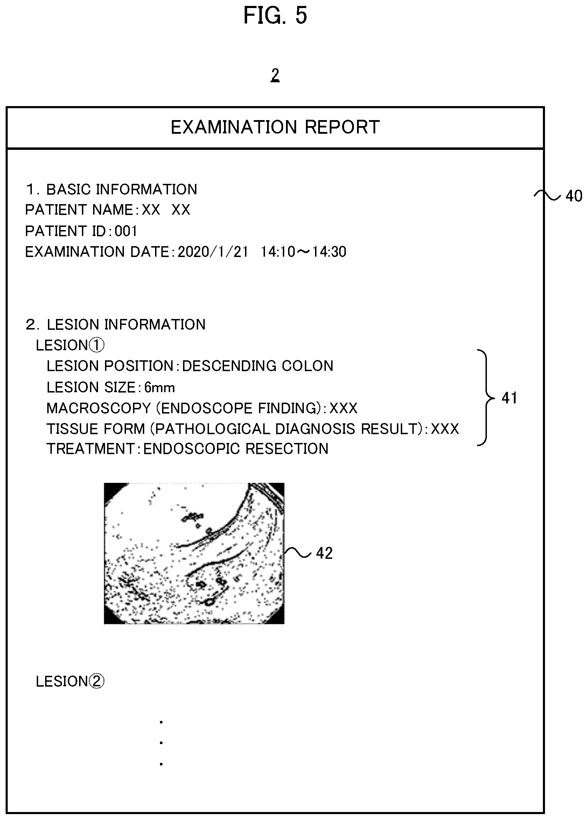

FIG. 5 shows a display example of the examination report. In this example, the representative image and information related to the lesion (hereinafter referred to as "related information") for a certain patient's lesion are displayed.

Specifically, in the display example of FIG. 5, the related information 41 and the lesion image 42 are displayed in the display area 40 in addition to the basic information such as the patient name, the patient ID, and the examination date and time. The related information 41 is information about the lesion included in the lesion image 42. The related information 41 includes the lesion position, the lesion size, the macroscopy (endoscope finding), the tissue form (pathological diagnosis result), and treatment, etc. The lesion image 42 is the representative image selected by the AI determination unit 23 from a plurality of images that are grouped as corresponding to the same lesion. That is, the representative image is the image that best represents the lesion from the plurality of grouped images. The related information 41 and the lesion image 42 are displayed for each detected lesion. Incidentally, the lesion thus displayed may be the image captured by the examiner and may be the image captured automatically by the AI determination unit 23.

Thus, in the present example embodiment, the image processing device 1 selects the representative image from among the plurality of images corresponding to the same lesion captured in the endoscopic examination and attaches the representative image to the examination report. This eliminates the labor for the examiner to select the image, and makes it possible to create the examination report efficiently.

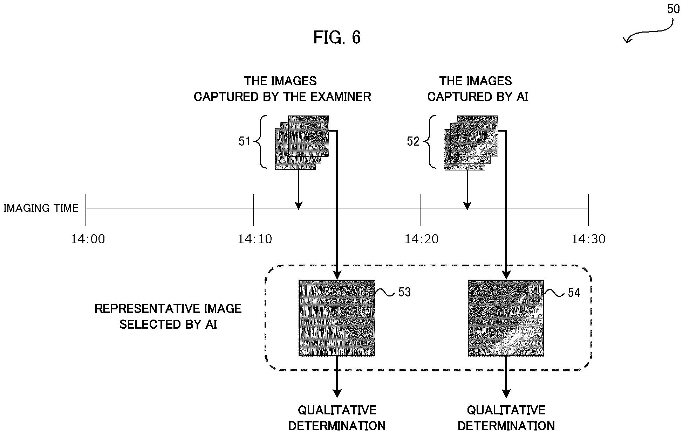

FIG. 6 shows another display example of the examination report. In this example, a plurality of still images determined to be the same lesion and the representative image selected from the plurality of still images are displayed in association with the imaging time of the endoscopic image Ic. Incidentally, the display example of FIG. 6 is used in addition to the examination report shown in FIG. 5, if necessary. In this instance, there are a plurality of images captured by the examiner between 14:10 and 14:20 and a plurality of images captured by AI between 14:20 and 14:30 in an endoscopic examination of a certain patient. At this time, in the display area 50, the plurality of still images and the representative image selected from the plurality of still images are displayed on the timeline indicating the elapsed time of the examination.

Specifically, the image processing device 1 groups the images corresponding to the same lesion for the images captured between 14:10 and 14:20. The image processing device 1 displays the grouped image group 51 and the representative image 53 selected from the image group 51 on the timeline. In addition, the image processing device 1 groups the images corresponding to the same lesion for the images captured between 14:20 and 14:30. The image processing device 1 displays the grouped image group 52 and the representative image 54 selected from the image group 52 on the timeline. Thus, by displaying the representative image in a manner associated with the image group corresponding to the imaging time and the same lesion, the examiner can easily grasp the image captured by himself and the image captured by AI in chronological order.

### [Image display processing]

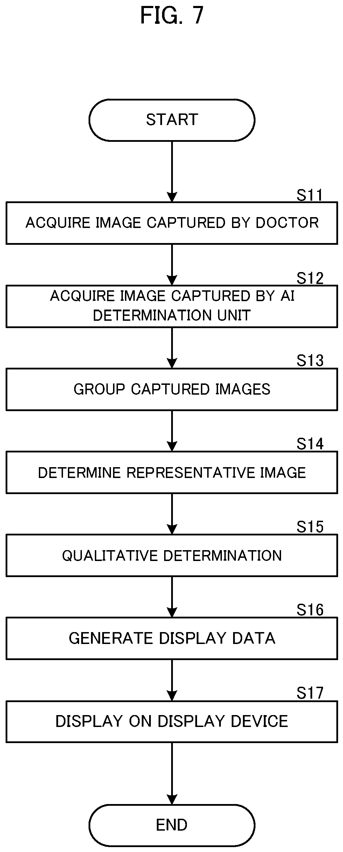

Next, display processing for performing the above-mentioned display will be described. FIG. 7 is a flowchart of display processing by the image processing device 1. This processing is realized by the processor 11 shown in FIG. 2, which executes a pre-prepared program and operates as each element shown in FIG. 3.

First, the examination data generation unit 22 acquires the lesion images captured by the doctor (step S 11). Further, the AI determination unit 23 captures the lesion image by itself on the basis of the endoscopic image Ic and the examination data generation unit 22 acquires the image captured by the AI determination unit 23 (step S12). Incidentally, step S12 may be performed prior to step S11, or may be performed simultaneously with step S11.

Next, the AI determination unit 23 groups the images related to the same lesion from the plurality of lesion images captured within a predetermined period (step S13). Next, the AI determination unit 23 determines the representative image which best represents the lesion from the grouped image group (step S14). Furthermore, the AI determination unit 23 performs qualitative determination for the lesion of the representative image (step S15).

Next, the display data generation unit 24 generates the display data as illustrated in FIG. 5 using the examination data generated by the examination data generation unit 22, the representative image determined by the AI determination unit 23, and the qualitative determination result of the AI determination unit 23, and outputs the display data to the display device 2 (step S16). The display device 2 displays the received the display data (step S17). Thus, the display like the display example of FIG. 5 is performed.

### <Second example embodiments

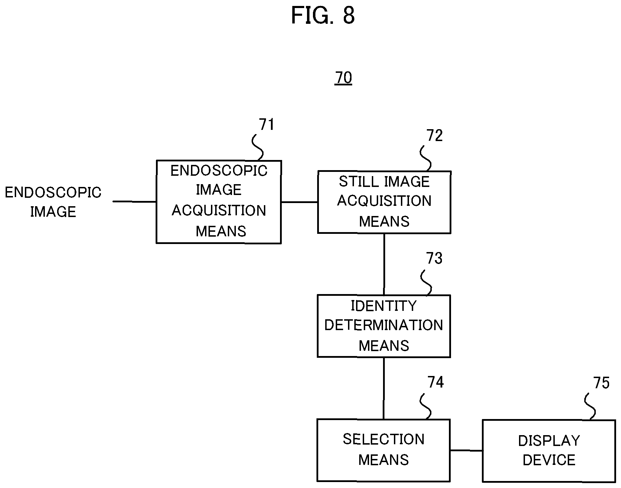

FIG. 8 is a block diagram showing a functional configuration of an information processing device of a second example embodiment. The information processing device 70 includes an endoscopic image acquisition means 71, a still image acquisition means 72, an identity determination means 73, a selection means 74, and a display device 75.

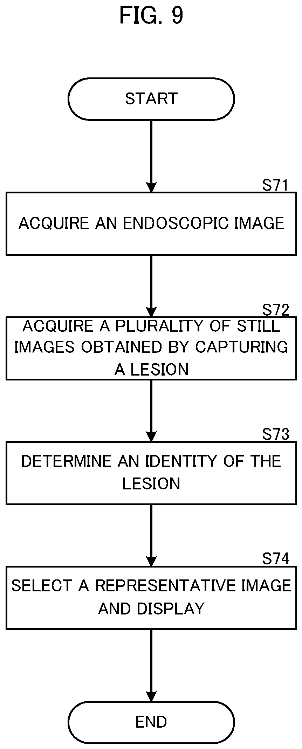

FIG. 9 is a flowchart of processing by the information processing device of the second example embodiment. The endoscopic image acquisition means 71 acquires an endoscopic image (step S71). The still image acquisition means 72 acquires a plurality of still images obtained by imaging a lesion included in the endoscopic image (step S72). The identity determination means 73 determines an identity of the lesion included in the plurality of still images (step S73). The selection means 74 selects a representative image that best represents the lesion, from a plurality of still images determined to correspond to the same lesion, and displays the representative image on the display device 75 (step S74).

According to the information processing device 70 of the second example embodiment, it is possible to save time and effort for selecting the image to be attached to the examination report by the examiner, and efficiently create the examination report.

A part or all of the above example embodiments may also be described as in the following supplementary notes, but are not limited to:

### (Supplementary note 1)

An information processing device comprising:
an endoscopic image acquisition means configured to acquire an endoscopic image;
a still image acquisition means configured to acquire a plurality of still images obtained by imaging a lesion included in the endoscopic image;
an identity determination means configured to determine an identity of the lesion included in the plurality of still images; and
a selection means configured to select a representative image that best represents the lesion, from a plurality of still images determined to correspond to the same lesion.

### (Supplementary note 2)

The information processing device according to Supplementary note 1, wherein the representative image is a still image in which the lesion is the largest among the plurality of still images that are determined to correspond to the same lesion.

### (Supplementary note 3)

The information processing device according to Supplementary note 1, wherein the representative image is a still image in which the lesion is the most centered among a plurality of still images that are determined to correspond to the same lesion.

### (Supplementary note 4)

The information processing device according to Supplementary note 1, wherein the representative image is a still image which is the most in focus among the plurality of still images that are determined to correspond to the same lesion.

### (Supplementary note 5)

The information processing device according to Supplementary note 1, further comprising a determination means configured to determine lesion likelihood for the plurality of still images,
wherein the representative image is a still image which is determined to be most likely to be a lesion by the determination means.

### (Supplementary note 6)

The information processing device according to any one of Supplementary notes 1 to 5, further comprising a display means configured to display an image in which the plurality of still images determined to correspond to the same lesion and the representative image selected from the plurality of still images are shown in association with an imaging time of the endoscopic image.

### (Supplementary note 7)

The information processing device according to any one of Supplementary notes 1 to 6, wherein the plurality of still images includes still images captured on the basis of a photographing instruction of an examiner.

### (Supplementary note 8)

The information processing device according to any one of Supplementary notes 1 to 6, wherein the plurality of still images includes still images captured automatically by an AI imaging means that detects and captures the lesion in the endoscopic image.

### (Supplementary note 9)

An information processing method comprising:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.

### (Supplementary note 10)

A recording medium storing a program, the program causing a computer to execute processing of:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
17 Database (DB)
21 Position detection unit
22 Examination data generation unit
23 AI determination unit
24 Display data generation unit
100 Endoscopic examination system

## Claims

1. An information processing device comprising:
an endoscopic image acquisition means configured to acquire an endoscopic image;
a still image acquisition means configured to acquire a plurality of still images obtained by imaging a lesion included in the endoscopic image;
an identity determination means configured to determine an identity of the lesion included in the plurality of still images; and
a selection means configured to select a representative image that best represents the lesion, from a plurality of still images determined to correspond to the same lesion.

2. The information processing device according to claim 1, wherein the representative image is a still image in which the lesion is the largest among the plurality of still images that are determined to correspond to the same lesion.

3. The information processing device according to claim 1, wherein the representative image is a still image in which the lesion is the most centered among a plurality of still images that are determined to correspond to the same lesion.

4. The information processing device according to claim 1, wherein the representative image is a still image which is the most in focus among the plurality of still images that are determined to correspond to the same lesion.

5. The information processing device according to claim 1, further comprising a determination means configured to determine lesion likelihood for the plurality of still images,
wherein the representative image is a still image which is determined to be most likely to be a lesion by the determination means.

6. The information processing device according to any one of claim 1 to 5, further comprising a display means configured to display an image in which the plurality of still images determined to correspond to the same lesion and the representative image selected from the plurality of still images are shown in association with an imaging time of the endoscopic image.

7. The information processing device according to any one of claim 1 to 6, wherein the plurality of still images includes still images captured on the basis of a photographing instruction of an examiner.

8. The information processing device according to any one of claims 1 to 6, wherein the plurality of still images includes still images captured automatically by an AI imaging means that detects and captures the lesion in the endoscopic image.

9. An information processing method comprising:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.

10. A recording medium storing a program, the program causing a computer to execute processing of:
acquiring an endoscopic image;
acquiring a plurality of still images obtained by imaging a lesion included in the endoscopic image;
determining an identity of the lesion included in the plurality of still images; and
selecting a representative image that best represents the lesion, from a plurality of still images determined to correspond to the same lesion.
